# EUROPEAN PATENT SPECIFICATION

(11) **EP 4 201 540 B1**
(45) Date of publication and mention of the grant of the patent: **26.08.2026**
(21) Application number: 21872419.3
(22) Date of filing: 21.09.2021
(51) Int. Cl.: B09B 3/60, B09B 3/40

(54) **WASTE TREATMENT SYSTEM**
ABFALLBEHANDLUNGSSYSTEM
SYSTÈME DE TRAITEMENT DE DÉCHETS

(30) Priority: 28.09.2020 JP 2020162457
(43) Date of publication of application: 28.06.2023
(73) Proprietor: Mitsubishi Heavy Industries, Ltd., Tokyo 100-8332 (JP)
(72) Inventor: KAWAI, Kazuhiro, Tokyo 100-8332 (JP); NOMA, Akira, Tokyo 100-8332 (JP); FUJIKAWA, Keiji, Tokyo 100-8332 (JP); OKINO, Susumu, Tokyo 100-8332 (JP)
(74) Representative: Studio Torta S.p.A.
(86) International application number: PCT/JP2021/034535
(87) International publication number: WO 2022/065290

(56) References cited:
- CN-A- 105 396 862
- JP-A- 2004 525 756
- JP-A- 2009 119 378
- JP-A- 2011 240 253
- JP-A- 2011 516 246
- JP-A- 2016 028 800
- JP-A- 2020 138 121
- JP-A- S6 082 197

## Description

### TECHNICAL FIELD

The present disclosure relates to a waste treatment system and a waste treatment method.

### BACKGROUND ART

It is desired to effectively use unused biomass, for example, urban waste from households, food factories, or the like, agricultural waste such as rice straw, wheat straw, and palm residue, livestock excreta, and sewage sludge. For example, in a technique described in Patent Document 1, waste is hydrothermally treated (hydrolyzed) with steam by a reformer, and solid fuel is produced from a solid phase of a reactant obtained by the hydrolysis. Further, combustion energy generated by combustion of the solid fuel is used to generate steam, and the generated steam is used to perform the hydrolysis.

### Citation List

### Patent Literature

Patent Document 1: JP2013-511386A (translation of a PCT application); and
Patent Document JP 2009 119378 A describing a methane fermentation method of organic waste.

### SUMMARY

### Problems to be Solved

However, in the technique described in Patent Document 1, since combustion energy generated by combustion of the solid fuel is used to generate steam, there is a risk that the demand for steam from the reformer cannot be met promptly, and the reformer may be put on standby.

The present disclosure was made in view of the above problem, and an object thereof is to provide a waste treatment system and a waste treatment method whereby it is possible to promptly meet the steam demand from the reformer.

### Solution to the Problems

In order to achieve the above object, a waste treatment system according to the present disclosure includes: at least one reformer for hydrolyzing waste with steam; a microbial reactor for microbially degrading a reformed material containing at least a solid of the waste hydrolyzed by the at least one reformer; and at least one steam generation device for generating the steam by using only combustion energy of a gas produced in the microbial reactor.

In order to achieve the above object, a waste treatment method according to the present disclosure includes: a step of hydrolyzing waste with steam; a step of microbially degrading a reformed material containing at least a solid of the hydrolyzed waste; and a step of generating the steam by using only combustion energy of a gas produced in the step of microbially degrading the reformed material.

### Advantageous Effects

With the waste treatment system and the waste treatment method of the present disclosure, it is possible to promptly meet the steam demand from the reformer.

### BRIEF DESCRIPTION OF DRAWINGS

FIG. 1 is a schematic configuration diagram of the waste treatment system according to the first embodiment of the present disclosure (embodiment not falling within the scope of the claims).
FIG. 2 is a schematic configuration diagram of the waste treatment system according to the second embodiment of the present disclosure (embodiment not falling within the scope of the claims).
FIG. 3 is a schematic configuration diagram of the waste treatment system according to the third embodiment of the present disclosure (embodiment not falling within the scope of the claims).
FIG. 4 is a schematic configuration diagram of the waste treatment system according to the fourth embodiment of the present disclosure covered by the attached claims.
FIG. 5 is a schematic configuration diagram of the waste treatment system according to the fifth embodiment of the present disclosure.
FIG. 6 is a partial schematic configuration diagram of the waste treatment system according to an embodiment of the present disclosure, where the surrounding configuration of the reformer is shown.
FIG. 7A is a graph showing a relationship between time and amount of steam supplied to the first reformer according to an embodiment of the present disclosure.
FIG. 7B is a graph showing a relationship between time and amount of steam supplied to the second reformer according to an embodiment of the present disclosure.
FIG. 8 is a flowchart of the waste treatment method according to an embodiment of the present disclosure.

### DETAILED DESCRIPTION

Hereinafter, a waste treatment system and a waste treatment method according to embodiments of the present disclosure will be described with reference to the drawings.

### (First Embodiment - not falling within the scope of the claim)

### <Configuration of waste treatment system according to first embodiment>

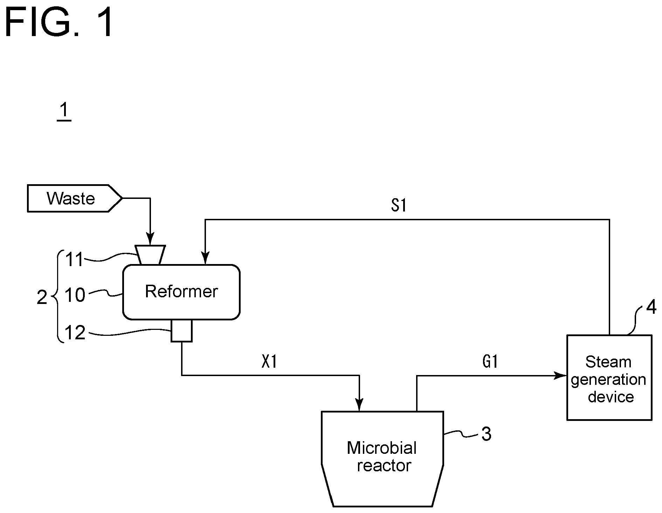

FIG. 1 is a schematic configuration diagram of the waste treatment system 1 according to the first embodiment of the present disclosure. As shown in FIG. 1, the waste treatment system 1 according to the first embodiment of the present disclosure includes a reformer 2, a microbial reactor 3, and a steam generation device 4.

The reformer 2 hydrolyzes waste with steam S1. The reformer 2 is configured to receive waste such as municipal waste as it is from a vehicle, a plant, or the like where the waste is collected, and hydrolyze the waste in a batch manner with steam S1, for example. Specifically, the reformer 2 is a batch-type reformer including a housing 10 with an input port 11 through which the waste is input and a discharge port 12 through which the hydrolyzed waste is discharged. The input port 11 and the discharge port 12 are provided with opening/closing valves (not shown), and the housing 10 can be sealed by closing the opening/closing valves. The hydrolysis of the waste in the reformer 2 may be wet hydrolysis in which steam S1 contacts the waste and heats the waste, or may be dry hydrolysis in which steam S1 indirectly heats the waste without contacting the waste. In the case of dry hydrolysis, moisture in the waste within the housing 10 evaporates into water vapor, and the water vapor uniformly heats the waste within the housing 10. Further, moisture required for hydrolysis is supplied as moisture from the water vapor adheres to the surface of the waste. Although one reformer 2 is shown in FIG. 1, a plurality of reformers 2 may be connected in series, or a plurality of reformers 2 may be connected in parallel, or the configuration where a plurality of reformers 2 are connected in series and the configuration where a plurality of reformers 2 are connected in parallel may be combined.

The municipal waste, which is given as an example of the waste, mainly contains kitchen waste, paper waste, and plastic waste, with a small amount of metal. The waste to be treated by the waste treatment system 1 is not limited to municipal waste. The waste treatment system 1 can also treat waste such as sludge generated by treating wastewater from factories or the like and agricultural waste with a higher moisture content than municipal waste.

The microbial reactor 3 microbially degrades a reformed material X1 containing at least a solid of the waste hydrolyzed by the reformer 2. In this process, the microbial reactor 3 produces biogas G1. The microbial reactor 3 may have any configuration as long as it uses the reformed material X1 as a raw material and utilizes the biological action of microorganisms to produce biogas G1. For example, the microbial reactor 3 may be a biogas fermenter for producing biogas G1 such as methane as valuables. However, the microbial reactor 3 is not limited to the biogas fermenter. For example, the microbial reactor 3 may be a saccharification tank for producing sugar as valuables from carbohydrates such as starch and cellulose, or a composting device for producing compost by composting. With the configuration illustrated in FIG. 1, since the microbial reactor 3 produces valuables by microbially degrading the waste hydrolyzed by the reformer 2 without solidliquid separation, the waste treatment system 1 can treat even waste with low moisture content at low cost.

The steam generation device 4 generates steam S1 by using only combustion energy of biogas G1 produced in the microbial reactor 3. In other words, the steam generation device 4 does not generate steam S1 by combustion energy of fuel other than biogas G1 produced in the microbial reactor 3. Then, the reformer 2 hydrolyzes waste with steam S1 generated by the steam generation device 4. Such a steam generation device 4 includes, for example, an exhaust gas boiler. The exhaust gas boiler generates steam S1 by heat of exhaust gas generated by combustion of biogas G1. The steam generation device 4 is not limited to the exhaust gas boiler. The steam generation device 4 may include a combustion boiler for generating steam S1 by using biogas G1 as fuel, instead of the exhaust gas boiler. Alternatively, the steam generation device 4 may include both the exhaust gas boiler and the combustion boiler.

### <Effect of waste treatment system according to first embodiment- not falling within the scope of the claims >

The effect of the waste treatment system 1 according to the first embodiment of the present disclosure will be described. Gaseous fuels burn faster than solid or liquid fuels. According to the first embodiment, the reformer 2 performs hydrolysis with steam generated by using only combustion energy of biogas G1 produced in the microbial reactor 3. Thus, it is possible to promptly meet the demand for steam S1 from the reformer 2.

In the case where the reformer 2 hydrolyzes waste with low moisture content (e.g., municipal waste) to produce the reformed material X1, microbially degrading solid in the reformed material X1 can yield more biogas G1 than microbially degrading only liquid in the reformed material X1. According to the first embodiment, the microbial reactor 3 microbially degrades the reformed material X1 containing at least a solid of the waste hydrolyzed by the reformer 2. Thus, it is possible to increase the production amount of biogas G1.

### (Second Embodiment- not falling within the scope of the claims)

Next, the waste treatment system 1 according to the second embodiment will be described. The waste treatment system 1 according to the second embodiment is the addition of a heat retention steam pipe 8 to the first embodiment. In the second embodiment, the same constituent element as those in the first embodiment are associated with the same reference numerals and not described again in detail.

### <Configuration of waste treatment system according to second embodiment- not falling within the scope of the claims >

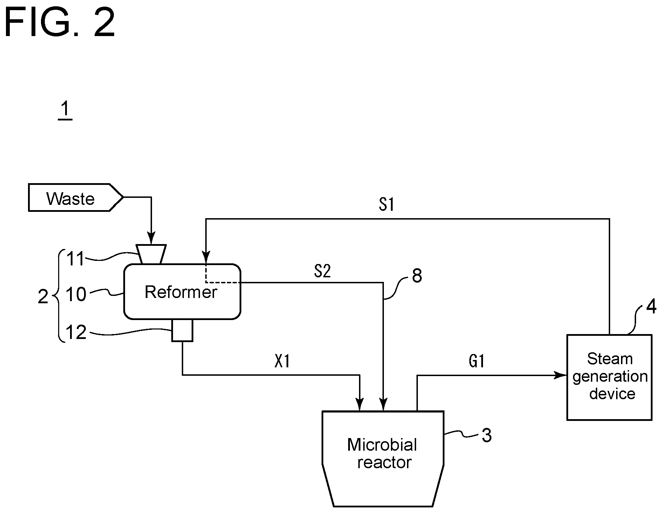

FIG. 2 is a schematic configuration diagram of the waste treatment system 1 according to the second embodiment of the present disclosure. As shown in FIG. 2, the waste treatment system 1 further includes a heat retention steam pipe 8 connecting the reformer 2 and the microbial reactor 3.

Exhaust steam S2 discharged from the reformer 2 after hydrolyzing the waste in the reformer 2 flows through the heat retention steam pipe 8. The reformer 2 supplies exhaust steam S2 to the microbial reactor 3 through the heat retention steam pipe 8. The microbial reactor 3 is kept within a predetermined temperature range, for example, between 30 degrees and 50 degrees, by heat of exhaust steam S2 supplied from the reformer 2.

### <Effect of waste treatment system according to second embodiment- not falling within the scope of the claims >

The microbial reactor 3 is desirably kept within a predetermined temperature range in order to promote the microbial degradation of the reformed material X1. According to the second embodiment, exhaust steam S2 is used as a heat source for heat retention of the microbial reactor 3. Thus, the amount of fuel prepared for heat retention of the microbial reactor 3 can be suppressed, and the operating cost can be reduced.

### (Third Embodiment- not falling within the scope of the claims)

Next, the waste treatment system 1 according to the third embodiment will be described. The waste treatment system 1 according to the third embodiment is the addition of a drying device 13 and a drying steam pipe 15 to the first embodiment. In the third embodiment, the same constituent elements as those in the first embodiment are associated with the same reference numerals and not described again in detail. The waste treatment system 1 according to another embodiment may be the addition of a drying device 13 and a drying steam pipe 15 to the second embodiment.

### <Configuration of waste treatment system according to third embodiment - not falling within the scope of the claims >

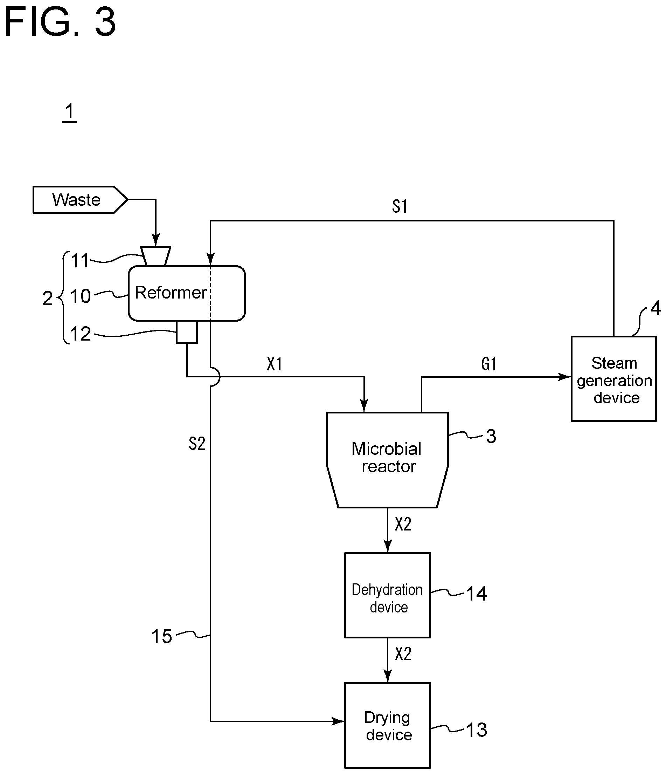

FIG. 3 is a schematic configuration diagram of the waste treatment system 1 according to the third embodiment of the present disclosure. As shown in FIG. 3, the waste treatment system 1 further includes a drying device 13 for drying a residue X2 obtained after the microbial reactor 3 microbially degrades the reformed material X1, and a drying steam pipe 15 connecting the reformer 2 and the drying device 13.

In the embodiment illustrated in FIG. 3, the waste treatment system 1 includes a dehydration device 14 for dehydrating the residue X2 (fermentation residue) that remains after the microbial reactor 3 microbially degrades the reformed material X1. The drying device 13 is supplied with the residue X2 dehydrated by the dehydration device 14.

Exhaust steam S2 discharged from the reformer 2 after hydrolyzing the waste in the reformer 2 flows through the drying steam pipe 15. The reformer 2 supplies exhaust steam S2 to the drying device 13 through the drying steam pipe 15. The drying device 13 dries the residue X2 dehydrated by the dehydration device 14 by heat of exhaust steam S2 supplied from the reformer 2.

### <Effect of waste treatment system according to third embodiment- not falling within the scope of the claims >

As illustrated in FIG. 3, the waste treatment system 1 with the microbial reactor 3 may include the drying device 13 for drying the residue X2 obtained from the microbial reactor 3. According to the third embodiment, exhaust steam S2 is used as a heat source for drying the residue X2 by the drying device 13. Thus, the amount of fuel prepared for drying the residue X2 by the drying device 13 can be suppressed, and the operating cost can be reduced.

### (Fourth Embodiment)

### <Configuration of waste treatment system according to fourth embodiment>

Next, the waste treatment system 1 according to the fourth embodiment will be described. The waste treatment system 1 according to the fourth embodiment may be the addition of a separation device 16 to the first embodiment. In the fourth embodiment, the same constituent elements as those in the first embodiment are associated with the same reference numerals and not described again in detail. The waste treatment system 1 according to another embodiment may be the addition of a separation device 16 to the second or third embodiment.

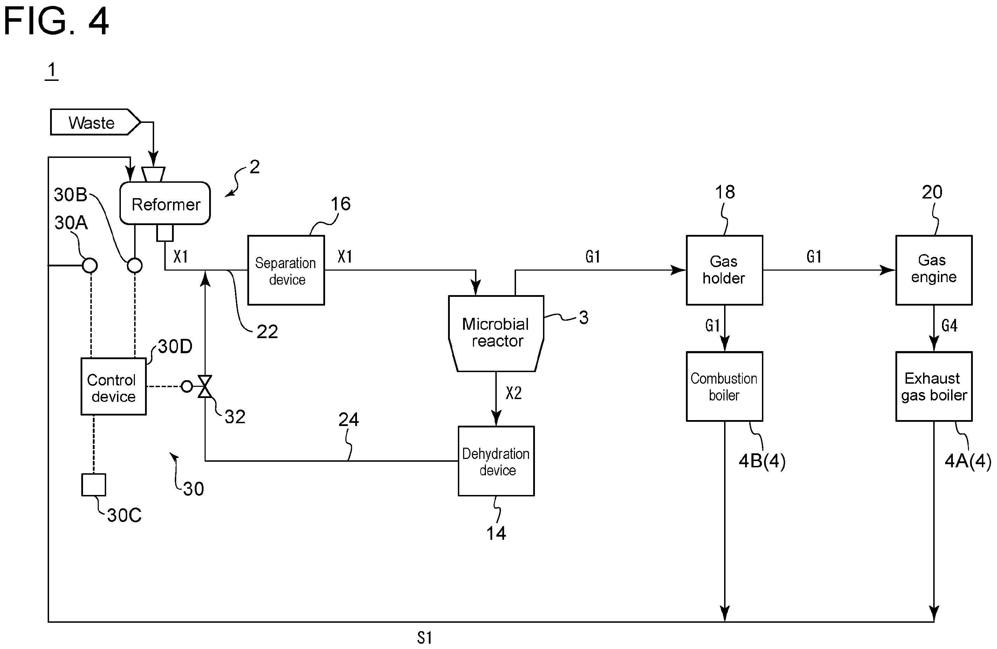

FIG. 4 is a schematic configuration diagram of the waste treatment system 1 according to the fourth embodiment of the present disclosure. As shown in FIG. 4, the waste treatment system 1 further includes a separation device 16 disposed between the reformer 2 and the microbial reactor 3.

The separation device 16 separates an unsuitable substance that is unsuitable for producing biogas G1 in the microbial reactor 3 from the reformed material X1 obtained by hydrolyzing the waste in the reformer 2. The separation device 16 serves to separate the reformed material X1 into a large particle size component and a small particle size component having a smaller particle size than the large particle size component. The separation device 16 is, for example, a screen having any mesh size, which corresponds to a particle size at a boundary between the large particle size component and the small particle size component. The small particle size component is degraded in the microbial reactor 3 to produce valuables. The large particle size component is separated from the reformed material X1 and is not supplied to the microbial reactor 3. A principal component of the large particle size component has a relatively large particle size even after hydrolysis in the reformer 2, and cannot be degraded in the microbial reactor 3, such as those derived from plastic waste or metal. To put it another way, the large particle size component and the small particle size component are, respectively, a reaction-unsuitable substance and a reaction-suitable substance for microbial reaction.

In the embodiment illustrated in FIG. 4, the waste treatment system 1 includes the above-described dehydration device 14, a reformed material transfer line 22 connecting the reformer 2 and the separation device 16, and a water injection pipe 24 connecting the dehydration device 14 and the reformed material transfer line 22.

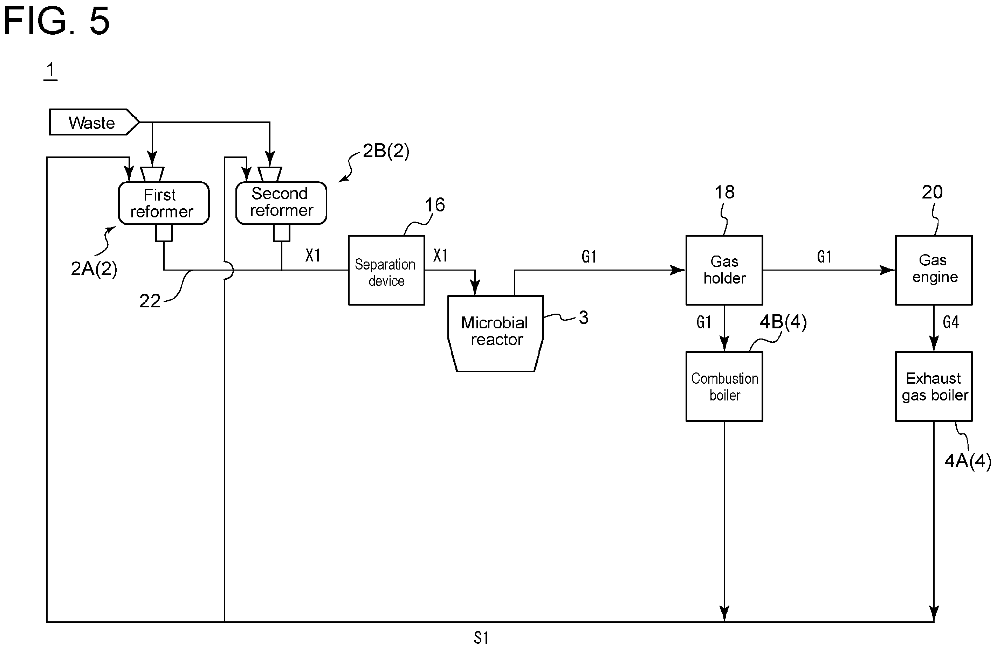

The reformed material transfer line 22 may be a pipe if the reformed material X1 is slurry, or may be a conveyor or the like if the reformed material X1 is solid. Even if the reformed material X1 is solid, as long as the reformed material X1 can be pumped by air or the like, the reformed material transfer line 22 may be a pipe. The dehydration device 14 supplies water dehydrated from the residue X2 of the microbial reactor 3 or boiler blowdown to the reformed material X1 in the reformed material transfer line 22 through the water injection pipe 24. In other words, the water injection pipe 24 is configured as a moisture adjustment device, disposed between the reformer 2 and the microbial reactor 3, for adjusting the moisture content of the reformed material X1. In the embodiment illustrated in FIG. 5, the water injection pipe 24 adjusts the moisture content of the reformed material X1 upstream of the separation device 16. In another embodiment, the water injection pipe 24 may adjust the moisture content of the reformed material X1 downstream of the separation device 16.

In the embodiment illustrated in FIG. 4, the waste treatment system 1 further includes a moisture amount acquisition device 30 for acquiring the amount of moisture contained in the reformer 2. The moisture amount acquisition device 30 acquires the moisture amount contained in the reformer 2, based on statistical data or weight of the raw material supplied to the reformer 2 (including waste properties estimated by machine learning, AI, etc. based on images), consolidation torque, reformer temperature (inside, surface), amount of steam S1 supplied to the reformer 2, time during which steam S1 is supplied to the reformer 2, and amount of temperature change in the reformer 2. That is, the moisture amount acquisition device 30 includes a flow meter 30A for acquiring the amount of steam S1 supplied to the reformer 2, a thermometer 30B for acquiring the temperature in the reformer 2, a timer 30C, and a control device 30D. The control device 30D is electrically connected to each of the flow meter 30A, the thermometer 30B, and the timer 30C, and calculates (acquires) the moisture amount contained in the reformer 2, based on data acquired by each of the flow meter 30A, the thermometer 30B, and the timer 30C.

The moisture amount acquisition device 30 is not limited to the embodiment illustrated in FIG. 4 as long as it can acquire the moisture amount in the reformer 2. For example, the moisture amount acquisition device 30 may be a near-infrared, microwave, or capacitance moisture meter.

The water injection pipe 24 (moisture adjustment device) adjusts the moisture content of the reformed material X1 based on the moisture amount acquired by the moisture amount acquisition device 30. In the embodiment illustrated in FIG. 4, the water injection pipe 24 is provided with a regulating valve 32 for adjusting the amount of water (water dehydrated from the residue X2 of the microbial reactor 3 or boiler blowdown) supplied from the dehydration device 14 to the reformed material transfer line 22. The regulating valve 32 is electrically connected to the control device 30D, and the opening degree thereof is adjusted according to instructions from the control device 30D.

In the embodiment illustrated in FIG. 4, the waste treatment system 1 includes a gas holder 18 for storing biogas G1 produced in the microbial reactor 3, a gas engine 20 driven with biogas G1 stored in the gas holder 18 as fuel, an exhaust gas boiler 4A (4) for generating steam S1 by heat of exhaust gas G4 from the gas engine 20, and a combustion boiler 4B (4) for generating steam S1 with biogas G1 stored in the gas holder 18 as fuel.

Biogas G1 stored in the gas holder 18 is supplied to the gas engine 20. The gas engine 20 is connected to a generator body part (power generation device) (not shown). The generator body part generates power by using combustion energy generated by combustion of biogas G1. In this process, the gas engine 20 emits exhaust gas G4.

Exhaust gas G4 from the gas engine 20 is supplied to the exhaust gas boiler 4A. The exhaust gas boiler 4A (first steam generation device) serves to generate steam S1 supplied to the reformer 2. The exhaust gas boiler 4A generates steam S1 by using only combustion energy of biogas G1, i.e., heat of exhaust gas G4.

Biogas G1 stored in the gas holder 18 is also supplied to the combustion boiler 4B (second steam generation device). The combustion boiler 4B serves to generate steam S1 supplied to the reformer 2. The combustion boiler 4B uses biogas G1 as fuel and generates steam S1 by using only combustion energy of biogas G1.

### <Effect of waste treatment system according to fourth embodiment>

According to the fourth embodiment, since the separation device 16 is disposed between the reformer 2 and the microbial reactor 3, it is possible to reduce the amount of the unsuitable substance supplied to the microbial reactor 3. As a result, it is possible to reduce the risk of inhibiting production of biogas G1 in the microbial reactor 3, and to efficiently produce biogas G1 in the microbial reactor 3.

Additionally, according to the fourth embodiment, since the water injection pipe 24 functioning as the moisture adjustment device is disposed between the reformer 2 and the separation device 16, the total solid concentration (TS) of the reformed material X1 obtained by hydrolysis in the reformer 2 can be adjusted to improve the efficiency of production of biogas G1 in the microbial reactor 3. Further, since water obtained by dehydration of the residue X2 of the microbial reactor 3 contains ammonia, if the reformed material X1 has low nitrogen content, the reformed material X1 can also be replenished with a nitrogen-containing substance.

According to the findings of the present inventors, it has been found that the moisture amount in the reformer 2 can be obtained with high accuracy from the amount of steam S1 supplied to the reformer 2, time during which steam S1 is supplied to the reformer 2, and amount of temperature change in the reformer 2. According to the fourth embodiment, since the waste treatment system 1 further includes the moisture amount acquisition device 30 for acquiring the moisture amount contained in the reformer 2, the moisture amount contained in the reformer 2 can be obtained with high accuracy, so it is possible to further improve the efficiency of production of biogas G1 in the microbial reactor 3.

According to the fourth embodiment, since the total solid concentration (TS) of the reformed material X1 is adjusted based on the moisture amount contained in the reformer 2 acquired by the moisture amount acquisition device 30 (calculated by the control device 30D), it is possible to further improve the efficiency of production of biogas G1 in the microbial reactor 3.

The exhaust gas boiler 4A and the combustion boiler 4B do not necessarily have the same capacity to generate steam S1. Steam S1 is generated in at least one of the exhaust gas boiler 4A or the combustion boiler 4B according to the steam demand from the reformer 2. The steam demand from the reformer 2 is calculated, for example, based on the amount of waste fed into the reformer 2 and the moisture content of the waste.

If the steam demand from the reformer 2 can be met by the supply of steam S1 generated in one of the exhaust gas boiler 4A or the combustion boiler 4B, the other of the exhaust gas boiler 4A or the combustion boiler 4B may be put on standby, or steam may be generated for purposes other than supply to the reformer 2. If it is difficult to meet the steam demand from the reformer 2 by only the supply of steam S1 generated in one of the exhaust gas boiler 4A or the combustion boiler 4B, the shortage may be made up with steam S1 generated in the other of the exhaust gas boiler 4A or the combustion boiler 4B. Thus, efficient operation of the waste treatment system 1 can be realized.

Although the operation method for each of the exhaust gas boiler 4A and the combustion boiler 4B is not particularly limited, an example of the operation method will be described. While the waste is hydrolyzed in the reformer 2, the exhaust gas boiler 4A is always operated. If the hydrolysis conditions in the reformer 2 can be adjusted only by steam S1 generated in the exhaust gas boiler 4A, the combustion boiler 4B is not operated. When the hydrolysis conditions are adjusted according to the amount of waste fed to the reformer 2 or the composition of waste components, if steam S1 generated in the exhaust gas boiler 4A alone is insufficient, the combustion boiler 4B is operated to supply not only steam S1 generated in the exhaust gas boiler 4A but also steam S1 generated in the combustion boiler 4B to the reformer 2. Thus, it is possible to extend the adjustment range of hydrolysis conditions in the reformer 2, compared to the case where only the exhaust gas boiler 4A is provided. In addition, it is possible to further extend the adjustment range of hydrolysis conditions in the reformer 2 by providing two or more gas engines 20 and operating the number of gas engines 20 appropriate to the amount of steam S1 generated in the exhaust gas boiler 4A.

### (Fifth Embodiment)

### <Configuration of waste treatment system according to fifth embodiment>

Next, the waste treatment system 1 according to the fifth embodiment will be described. The waste treatment system 1 according to the fifth embodiment is limited to the case where a plurality of reformers 2 are provided, and differs from the first to fourth embodiments in this point. In the fifth embodiment, the same constituent element as those in the first to fourth embodiments are associated with the same reference numerals and not described again in detail.

FIG. 5 is a schematic configuration diagram of the waste treatment system 1 according to the fifth embodiment of the present disclosure. As shown in FIG. 5, the waste treatment system 1 further includes a first reformer 2A (2) and a second reformer 2B (2). In the embodiment illustrated in FIG. 5, the first reformer 2A (2) and the second reformer 2B (2) are connected in parallel with each other so that the destination of waste can be selected. The waste hydrolyzed by the first reformer 2A and the waste hydrolyzed by the second reformer 2B flow through a common reformed material transfer line 22 and are supplied to the separation device 16.

The first reformer 2A and the second reformer 2B hydrolyze the waste with steam S1 at different timings. For example, the timing of supplying steam S1 to the first reformer 2A and the timing of supplying steam S1 to the second reformer 2B are different from each other.

The number of reformers 2 installed in the waste treatment system 1 is not limited, but if the reformers 2 are batch reformers 2, the number of reformers 2 may be determined based on the batch period, which is a period from when waste is fed to the next time waste is fed, and the heating period, which is a period for raising the temperature in the reformer 2 to a predetermined set temperature with steam S1. Specifically, the number of reformers 2 is determined based on the natural number of values obtained by dividing the batch period by the heating period. With this configuration, it is possible to efficiently suppress changes in the demand for steam from the reformer 2.

### <Effect of waste treatment system according to fifth embodiment>

According to the fifth embodiment, compared to the case where the first reformer 2A and the second reformer 2B are hydrolyzed at an overlapping timing, the total steam demand to the exhaust gas boiler 4A and the combustion boiler 4B can be reduced, and the amount of steam S1 supplied to the first reformer 2A and the second reformer 2B can be prevented from becoming insufficient. Further, since the total steam demand required to the exhaust gas boiler 4A and the combustion boiler 4B is reduced, the amount of biogas G1 used for generating steam S1 can be reduced. Thus, by using the reduction in biogas G1 for power generation in the gas engine 20, the amount of power generated can be increased.

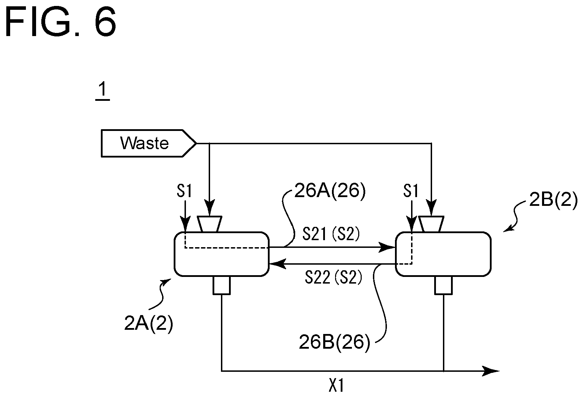

FIG. 6 is a partial schematic configuration diagram of the waste treatment system 1 according to an embodiment of the present disclosure, where the surrounding configuration of the reformer 2 is shown. In an embodiment, as shown in FIG. 6, the waste treatment system 1 further includes an exhaust steam distribution pipe 26 connecting the first reformer 2A and the second reformer 2B.

In the embodiment illustrated in FIG. 6, the exhaust steam distribution pipe 26 includes a first exhaust steam distribution pipe 26A and a second exhaust steam distribution pipe 26B. Exhaust steam S21 (S2) discharged from the first reformer 2A after hydrolyzing the waste in the first reformer 2A flows through the first exhaust steam distribution pipe 26A. The first reformer 2A supplies exhaust steam S21 to the second reformer 2B through the first exhaust steam distribution pipe 26A. The second reformer 2B is heated by heat of exhaust steam S21 supplied from the first reformer 2A.

Similarly, exhaust steam S22 (S2) discharged from the second reformer 2B after hydrolyzing the waste in the second reformer 2B flows through the second exhaust steam distribution pipe 26B. The second reformer 2B supplies exhaust steam S22 to the first reformer 2A through the second exhaust steam distribution pipe 26B. The first reformer 2A is heated by heat of exhaust steam S22 supplied from the second reformer 2B.

With the configuration illustrated in FIG. 6, exhaust steam S2 after hydrolyzing the waste in one reformer 2 is used as a heat source for heating the other reformer 2. Thus, the amount of fuel prepared for heating the other reformer 2 can be suppressed, and the operating cost can be reduced. In the embodiment illustrated in FIG. 6, the exhaust steam distribution pipe 26 includes the first exhaust steam distribution pipe 26A and the second exhaust steam distribution pipe 26B, but the present disclosure is not limited to this embodiment. The exhaust steam distribution pipe 26 may be configured to allow both exhaust steam S21 and exhaust steam S22 to flow. In other words, the first reformer 2A and the second reformer 2B may be configured to supply exhaust steam S2 to each other through the common exhaust steam distribution pipe 26.

In an embodiment, Tr/Tk ≥ 0.3 is satisfied, where Tr is a heating period for raising the temperature in the reformer 2 to a predetermined set temperature with steam S1, and Tk is a maintaining period during which the temperature in the reformer 2 is maintained at the set temperature with steam S1.

If the heating period Tr is short, in order to quickly raise the temperature in the reformer 2 to the set temperature, the steam demand required by the reformer 2 temporarily increases, so it may be difficult for the steam generation device 4 to meet the demand from the reformer 2. In contrast, satisfying Tr/Tk≥0.3 prevents the steam demand required by the reformer 2 from temporarily increasing, allowing the steam generation device 4 to meet the demand from the reformer 2.

In another embodiment, Tr/Tk ≥ 1.0 may be satisfied. With this configuration, since the heating period Tr is longer than the maintaining period Tk, it is possible to further suppress the temporarily increase in demand for steam from the reformer 2 and meet the demand from the reformer 2.

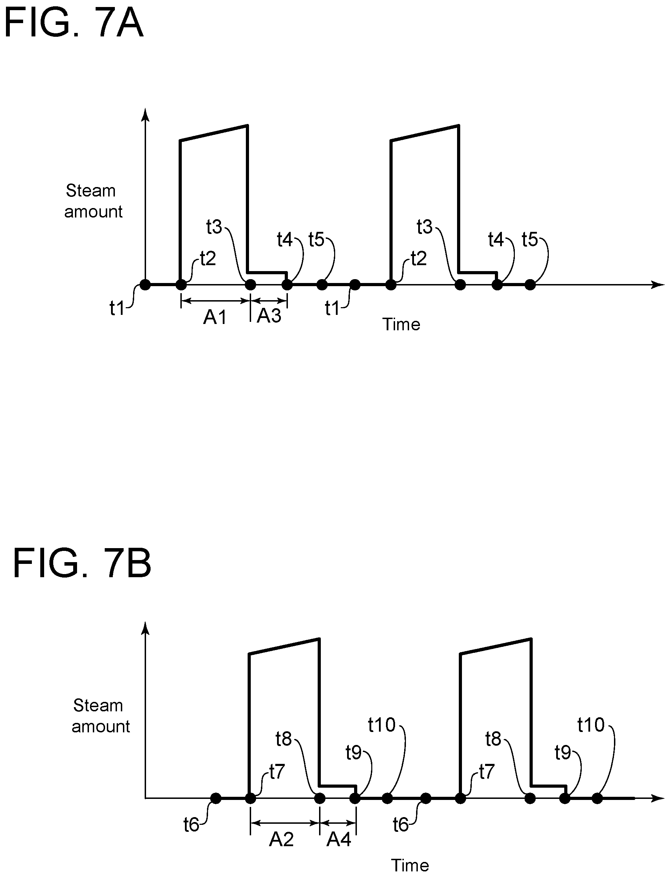

FIG. 7A is a graph showing a relationship between time and amount of steam S1 supplied to the first reformer 2A according to an embodiment. FIG. 7B is a graph showing a relationship between time and amount of steam S1 supplied to the second reformer 2B according to an embodiment. In FIGs. 7A and 7B, the horizontal axis represents time, and the vertical axis represents the amount of steam.

FIG. 7A will now be described. t1 is the timing to start feeding waste into the first reformer 2A. t2 is the timing to start supplying steam S1 to the first reformer 2A in order to raise the temperature in the first reformer 2A to the set temperature. At the timing of t2, the feeding of waste into the first reformer 2A has been completed. t3 is the timing to start supplying steam S1 to the first reformer 2A (with a small supply amount) in order to maintain the temperature in the first reformer 2A at the set temperature. t4 is the timing to start discharging steam S1 in the first reformer 2A to the outside. The waste is hydrolyzed in the first reformer 2A mainly between t2 and t4. t5 is the timing when the discharge of steam S1 in the first reformer 2A is completed. Once the discharge of steam S1 is completed, the first reformer 2A is placed on a standby mode until the next start of feeding waste (t1 for the second time).

FIG. 7B will now be described. t6 is the timing to start feeding waste into the second reformer 2B. t7 is the timing to start supplying steam S1 to the second reformer 2B in order to raise the temperature in the second reformer 2B to the set temperature. At the timing of t7, the feeding of waste into the second reformer 2B has been completed. t8 is the timing to start supplying steam S1 to the second reformer 2B (with a small supply amount) in order to maintain the temperature in the second reformer 2B at the set temperature. t9 is the timing to start discharging steam S1 in the second reformer 2B to the outside. The waste is hydrolyzed in the second reformer 2B mainly between t7 and t9. t10 is the timing when the discharge of steam S1 in the second reformer 2B is completed. Once the discharge of steam S1 is completed, the second reformer 2B is placed on a standby mode until the next start of feeding waste (t6 for the second time).

From t2 to t3, the amount of steam supplied to the first reformer 2A gradually increases. This is to prevent the temperature in the first reformer 2A from decreasing by the amount of heat dissipated by the first reformer 2A. Similarly, from t7 to t8, the amount of steam supplied to the second reformer 2B gradually increases.

The steam operation of the waste treatment system 1 according to an embodiment will be described with reference to both FIGs. 7A and 7B.

A1 is the first period for raising the temperature in the first reformer 2A to a predetermined set temperature with steam S1, and A2 is the second period for raising the temperature in the second reformer 2B to a predetermined set temperature with steam S1. That is, the first period A1 and the second period A2 correspond to the heating period Tr described above. In an embodiment, as shown in FIGs. 7A and 7B, the first reformer 2A is heated with steam S1 and the second reformer 2B is heated with steam S1 so that the first period A1 and the second period A2 do not overlap each other. With this configuration, compared to the case where the first period A1 and the second period A2 overlap each other, the total steam demand required to the exhaust gas boiler 4A and the combustion boiler 4B can be reduced.

A3 is the third period during which the temperature in the first reformer 2A is maintained at the set temperature with steam S1, and A4 is the fourth period during which the temperature in the second reformer 2B is maintained at the set temperature with steam S1. That is, the third period A3 and the fourth period A4 correspond to the maintaining period Tk described above. In an embodiment, the first reformer 2A is maintained at the set temperature with steam S1 while the second reformer 2B is heated with steam S1 so that the third period A3 is included within the second period A2. The amount of steam S1 to maintain the reformer 2 at the set temperature is very small compared to the amount of steam S1 to raise the temperature of the reformer 2 to the set temperature. In other words, even if steam operation is performed such that the first reformer 2A is maintained at the set temperature while the second reformer 2B is heated, there is little possibility of shortage of steam S1 supplied to the second reformer 2B. Therefore, the operating time of the first reformer 2A and the second reformer 2B can be increased. In another embodiment, the first reformer 2A may be heated with steam S1 while the second reformer 2B may be maintained at the set temperature with steam S1 so that the fourth period A4 is included within the first period A1.

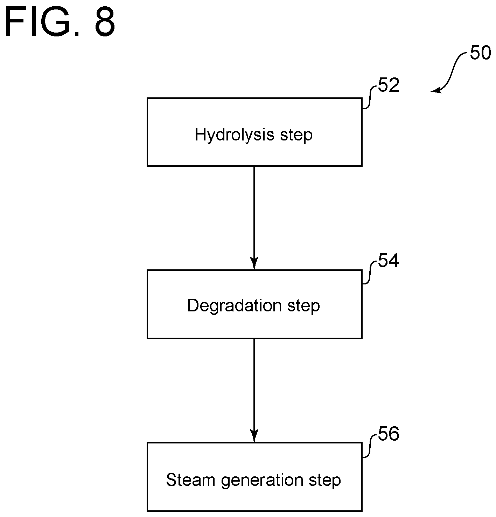

FIG. 8 is a flowchart of the waste treatment method 50 according to an embodiment of the present disclosure. As shown in FIG. 8, the waste treatment method 50 includes a step 52 of hydrolyzing waste with steam S1, a step 54 of microbially degrading a reformed material X1 containing at least a solid of the waste hydrolyzed with steam S1, and a step 56 of generating steam S1 by using only combustion energy of biogas G1 produced in the step 54 of microbially degrading the reformed material X1. With the method, it is possible to promptly meet the demand for steam S1 for hydrolyzing the waste.

The contents described in the above embodiments would be understood as follows, for instance. The following part is not a set of claims and it should not be interpreted as such.
[1] A waste treatment system (1) according to the present disclosure includes: at least one reformer (2) for hydrolyzing waste with steam (S1); a microbial reactor (3) for microbially degrading a reformed material (X1) containing at least a solid of the waste hydrolyzed by the at least one reformer; and at least one steam generation device (4) for generating the steam by using only combustion energy of a gas produced in the microbial reactor.
   Gaseous fuels burn faster than solid or liquid fuels. With the above configuration [1], the reformer performs hydrolysis with steam generated by using only combustion energy of the gas produced in the microbial reactor. Thus, it is possible to promptly meet the demand for steam from the reformer.
[2] In some embodiments, in the above configuration [1], the waste treatment system further includes a heat retention steam pipe (8) connecting the reformer and the microbial reactor. The reformer supplies exhaust steam (S2) after hydrolyzing the waste to the microbial reactor through the heat retention steam pipe. The microbial reactor is kept within a predetermined temperature range by heat of the exhaust steam supplied from the reformer.
   The microbial reactor is desirably kept within a predetermined temperature range in order to promote the microbial degradation of the reformed material. With the above configuration [2], exhaust steam after hydrolyzing the waste in the reformer is used as a heat source for heat retention of the microbial reactor. Thus, the amount of fuel prepared for heat retention of the microbial reactor can be suppressed, and the operating cost can be reduced.
[3] In some embodiments, in the above configuration [1] or [2], the waste treatment system further includes a drying device (13) for drying a residue (X2) obtained after the microbial reactor microbially degrades the reformed material; and a drying steam pipe (15) connecting the reformer and the drying device. The reformer supplies exhaust steam after hydrolyzing the waste to the drying device through the drying steam pipe. The drying device is configured to dry the residue by heat of the exhaust steam supplied from the reformer.
   The waste treatment system may include the drying device for drying the residue obtained from the microbial reactor. With the above configuration [3], exhaust steam after hydrolyzing the waste in the reformer is used as a heat source for drying the residue by the drying device. Thus, the amount of fuel prepared for drying the residue by the drying device can be suppressed, and the operating cost can be reduced.
[4] In some embodiments, in any one of the above configurations [1] to [3], the waste treatment system further includes a separation device (16) disposed between the at least one reformer and the microbial reactor. The separation device is configured to separate an unsuitable substance that is unsuitable for producing the gas in the microbial reactor from the waste hydrolyzed by the at least one reformer.
   With the above configuration [4], it is possible to reduce the amount of the unsuitable substance supplied to the microbial reactor. As a result, it is possible to reduce the risk of inhibiting production of the gas in the microbial reactor, and to efficiently produce the gas in the microbial reactor.
[5] In some embodiments, in any one of the above configurations [1] to [4], the at least one steam generation device includes a first steam generation device (4A) and a second steam generation device (4B). The waste treatment system is configured such that the steam is generated in at least one of the first steam generation device or the second steam generation device according to steam demand from the reformer.
   With the above configuration [5], if the steam demand from the reformer can be met by one of the first steam generation device or the second steam generation device, the other steam generation device may be put on standby, or steam may be generated for purposes other than supply to the reformer. If it is difficult to meet the steam demand from the reformer by one steam generation device alone, the shortage may be made up with steam generated in the other steam generation device. Thus, efficient operation of the waste treatment system can be realized.
[6] In some embodiments, in any one of the above configurations [1] to [5], the at least one reformer includes a plurality of reformers. At least two of the plurality of reformers are configured to perform hydrolysis at different timings.
   With the above configuration [6], compared to the case where at least two of the plurality of reformers are hydrolyzed at an overlapping timing, the total steam amount required to the steam generation device can be reduced, and the amount of steam supplied to the reformers can be prevented from becoming insufficient. Further, each of the reformers is supplied with steam generated by only combustion energy of the gas, not steam obtained by combusting solid or liquid fuel. Thus, it is possible to promptly meet the demand for steam from the reformer.
[7] In some embodiments, in the above configuration [6], the plurality of reformers includes a first reformer (2A) and a second reformer (2B). The waste treatment system further includes an exhaust steam distribution pipe (26) connecting the first reformer and the second reformer. Exhaust steam after hydrolyzing the waste is supplied from the first reformer to the second reformer through the exhaust steam distribution pipe. The second reformer is heated by heat of the exhaust steam supplied from the first reformer.
   With the above configuration [7], exhaust steam after hydrolyzing the waste in one reformer is used as a heat source for heating the other reformer. Thus, the amount of fuel prepared for heating the other reformer can be suppressed, and the operating cost can be reduced.
[8] In some embodiments, in any one of the above configurations [1] to [7], Tr/Tk ≥ 0.3 is satisfied, where Tr is a heating period for raising temperature in the reformer to a predetermined set temperature with the steam, and Tk is a maintaining period during which temperature in the reformer is maintained at the set temperature with the steam.
   If the heating period is short, in order to quickly raise the temperature in the reformer to the set temperature, the steam demand from the reformer temporarily increases, so it may be difficult to meet the demand from the reformer. In contrast, with the above configuration [8], since Tr/Tk≥0.3 is satisfied, the steam demand from the reformer is prevented from temporarily increasing, allowing to meet the demand from the reformer.
[9] In some embodiments, in the above configuration [8], Tr/Tk ≥ 1.0 is satisfied.
   With the above configuration [9], since the heating period is longer than the maintaining period, it is possible to further suppress the temporarily increase in demand for steam from the reformer and meet the demand from the reformer.
[10] In some embodiments, in any one of the above configurations [1] to [9], the waste treatment system further includes a moisture adjustment device (24), disposed between the at least one reformer and the microbial reactor, for adjusting moisture content of the waste hydrolyzed by the at least one reformer.
   With the above configuration [10], the total solid concentration (TS) of the waste hydrolyzed by the reformer can be adjusted to improve the efficiency of production of the gas by the microbial reactor.
[11] In some embodiments, in the above configuration [10], the waste treatment system further includes a moisture amount acquisition device (30) for acquiring moisture amount contained in the reformer. The moisture adjustment device is configured to adjust moisture content of the waste hydrolyzed by the at least one reformer, based on the moisture amount acquired by the moisture amount acquisition device.
   With the above configuration [11], since the total solid concentration (TS) of the waste hydrolyzed by the reformer is adjusted based on the moisture amount contained in the reformer, it is possible to further improve the efficiency of production of the gas by the microbial reactor.
[12] In some embodiments, in the above configuration [11], the moisture amount acquisition device is configured to acquire moisture amount contained in the reformer, based on the amount of steam supplied to the reformer, time during which the steam is supplied to the reformer, and amount of temperature change in the reformer.
   According to the findings of the present inventors, it has been found that the moisture amount in the reformer can be obtained with high accuracy from the amount of steam supplied to the reformer, time during which steam is supplied to the reformer, and amount of temperature change in the reformer. With the above configuration [12], it is possible to accurately acquire the moisture amount contained in the reformer, and further improve the efficiency of production of the gas by the microbial reactor.
[13] A waste treatment method (50) according to the present disclosure includes: a step (52) of hydrolyzing waste with steam; a step (54) of microbially degrading a reformed material containing at least a solid of the hydrolyzed waste; and a step (56) of generating the steam by using only combustion energy of a gas produced in the step of microbially degrading the reformed material.

With the above method [13], it is possible to promptly meet the demand for steam for hydrolyzing the waste.

### Reference Signs List

- 1: Waste treatment system
- 2: Reformer
- 2A: First reformer
- 2B: Second reformer
- 3: Microbial reactor
- 4: Steam generation device
- 4A: Exhaust gas boiler (First steam generation device)
- 4B: Combustion boiler (Second steam generation device)
- 8: Heat retention steam pipe
- 13: Drying device
- 15: Drying steam pipe
- 16: Separation device
- 24: Water injection pipe (Moisture adjustment device)
- 26: Exhaust steam distribution pipe
- 30: Moisture amount acquisition device
- 50: Waste treatment method
- A1: First period
- A2: Second period
- A3: Third period
- A4: Fourth period

- S1: Steam
- S2: Exhaust steam
- X1: Reformed material
- X2: Residue

## Claims

1. A waste treatment system (1), comprising:
at least one reformer (2) for hydrolyzing waste with steam (S1);
a microbial reactor (3) for microbially degrading a reformed material containing at least a solid of the waste hydrolyzed by the at least one reformer (2);
at least one steam generation device (4) for generating the steam (S1) by using only combustion energy of a gas produced in the microbial reactor (3);
a moisture amount acquisition device (30) for acquiring moisture amount contained in the reformer (2);
a moisture adjustment device (24), disposed between the at least one reformer (2) and the microbial reactor (3), for adjusting moisture content of the waste hydrolyzed by the at least one reformer (2), based on the moisture amount acquired by the moisture amount acquisition device (30);
a dehydration device (14) for dehydrating a residue (X2) that remains after the microbial reactor microbially degrades the reformed material,
wherein the moisture adjustment device (24) includes a water injection pipe (24) configured to supply dehydrated water, which is dehydrated from the residue by the dehydration device (14), to the reformed material while the reformed material is being sent from the reformer to the microbial reactor (3).

2. The waste treatment system (1) according to claim 1, further comprising a heat retention steam pipe (8) connecting the reformer (2) and the microbial reactor (3),
wherein exhaust steam after hydrolyzing the waste is supplied from the reformer (2) to the microbial reactor (3) through the heat retention steam pipe (8), and
wherein the microbial reactor (3) is kept within a predetermined temperature range by heat of the exhaust steam supplied from the reformer (2).

3. The waste treatment system (1) according to claim 1 or 2, further comprising:
a drying device (13) for drying a residue obtained after the microbial reactor (3) microbially degrades the reformed material; and
a drying steam pipe (15) connecting the reformer (2) and the drying device (13),
wherein exhaust steam after hydrolyzing the waste is supplied from the reformer (2) to the drying device (13) through the drying steam pipe (15), and
wherein the drying device (13) is configured to dry the residue by heat of the exhaust steam supplied from the reformer (2).

4. The waste treatment system (1) according to any one of claims 1 to 3, further comprising a separation device (16) disposed between the at least one reformer (2) and the microbial reactor (3),
wherein the separation device (16) is configured to separate an unsuitable substance that is unsuitable for producing the gas in the microbial reactor (3) from the waste hydrolyzed by the at least one reformer (2).

5. The waste treatment system (1) according to any one of claims 1 to 4,
wherein the at least one steam generation device (4) includes a first steam generation device (4A) and a second steam generation device (4B), and
wherein the waste treatment system (1) is configured such that the steam is generated in at least one of the first steam generation device (4A) or the second steam generation device (4B) according to steam demand from the reformer (2).

6. The waste treatment system (1) according to any one of claims 1 to 5,
wherein the at least one reformer (2) includes a plurality of reformers (2A, 2B), and
wherein at least two of the plurality of reformers (2A, 2B) are configured to perform hydrolysis at different timings.

7. The waste treatment system (1) according to claim 6,
wherein the plurality of reformers (2A, 2B) includes a first reformer (2A) and a second reformer (2B),
wherein the waste treatment system (1) further comprises an exhaust steam distribution pipe (26) connecting the first reformer (2A) and the second reformer (2B),
wherein exhaust steam after hydrolyzing the waste is supplied from the first reformer (2A) to the second reformer (2B) through the exhaust steam distribution pipe (26), and
wherein the second reformer (2B) is heated by heat of the exhaust steam supplied from the first reformer (2B).

8. The waste treatment system (1) according to any one of claims 1 to 7,
wherein Tr/Tk ≥ 0.3 is satisfied, where Tr is a heating period for raising temperature in the reformer (2) to a predetermined set temperature with the steam, and Tk is a maintaining period during which temperature in the reformer (2) is maintained at the set temperature with the steam (S1).

9. The waste treatment system (1) according to claim 8,
wherein Tr/Tk ≥ 1.0 is satisfied.

10. The waste treatment system (1) according to claim 1,
wherein the moisture amount acquisition device (30) is configured to acquire moisture amount contained in the reformer (2), based on the amount of steam supplied to the reformer (2), time during which the steam is supplied to the reformer (2), and amount of temperature change in the reformer (2).

11. The waste treatment system according to any one of claims 1 to 10, wherein the moisture adjustment device (24) further includes a regulating valve (32) provided in the water injection pipe (24), and wherein the regulating valve (32) is configured to have an opening degree adjusted based on the moisture amount acquired by the moisture amount acquisition device (30).

## Patentansprüche

1. Abfallbehandlungssystem (1), umfassend:
mindestens einen Reformer (2) zum Hydrolysieren von Abfall mit Dampf (S1);
einen mikrobiellen Reaktor (3) zum mikrobiellen Abbauen eines reformierten Materials, das mindestens einen Feststoff des durch den mindestens einen Reformer (2) hydrolysierten Abfalls enthält;
mindestens eine Dampferzeugungsvorrichtung (4) zum Erzeugen des Dampfs (S1) unter ausschließlicher Verwendung von Verbrennungsenergie eines in dem mikrobiellen Reaktor (3) gebildeten Gases;
eine Feuchtigkeitsmengenerfassungsvorrichtung (30) zum Erfassen einer in dem Reformer (2) enthaltenen Feuchtigkeitsmenge;
eine Feuchtigkeitsregulierungsvorrichtung (24), die zwischen dem mindestens einen Reformer (2) und dem mikrobiellen Reaktor (3) angeordnet ist, zum Regulieren eines Feuchtigkeitsgehalts des durch den mindestens einen Reformer (2) hydrolysierten Abfalls basierend auf der durch die Feuchtigkeitsmengenerfassungsvorrichtung (30) erfassten Feuchtigkeitsmenge;
eine Entwässerungsvorrichtung (14) zum Entwässern eines Rückstands (X2), der nach dem mikrobiellen Abbauen des reformierten Materials durch den mikrobiellen Reaktor verbleibt,
wobei die Feuchtigkeitsregulierungsvorrichtung (24) ein Wassereinspritzrohr (24) beinhaltet, das ausgelegt ist, um dem reformierten Material entwässertes Wasser zuzuführen, das durch die Entwässerungsvorrichtung (14) aus dem Rückstand entwässert ist, während das reformierte Material von dem Reformer zu dem mikrobiellen Reaktor (3) befördert wird.

2. Abfallbehandlungssystem (1) nach Anspruch 1, ferner umfassend ein Wärmerückhaltedampfrohr (8), das den Reformer (2) und den mikrobiellen Reaktor (3) verbindet,
wobei der Abdampf nach dem Hydrolysieren des Abfalls von dem Reformer (2) durch das Wärmerückhaltedampfohr (8) dem mikrobiellen Reaktor (3) zugeführt wird, und wobei der mikrobielle Reaktor (3) durch die Wärme des von dem Reformer (2) zugeführten Abdampfs innerhalb eines vorbestimmten Temperaturbereichs gehalten wird.

3. Abfallbehandlungssystem (1) nach Anspruch 1 oder 2, ferner umfassend:
eine Trocknungsvorrichtung (13) zum Trocknen eines nach dem mikrobiellen Abbauen des reformierten Materials (3) durch den mikrobiellen Reaktor erhaltenen Rückstands, und
ein Trocknungsdampfrohr (15), das den Reformer (2) und die Trocknungsvorrichtung (13) verbindet,
wobei der Abdampf nach dem Hydrolysieren des Abfalls von dem Reformer (2) durch das Trockungsdampfrohr (15) der Trocknungsvorrichtung (13) zugeführt wird, und
wobei die Trocknungsvorrichtung (13) ausgelegt ist, um den Rückstand durch die Wärme des von dem Reformer (2) zugeführten Abdampfs zu trocknen.

4. Abfallbehandlungssystem (1) nach einem der Ansprüche 1 bis 3, ferner umfassend eine Trennvorrichtung (16), die zwischen dem mindestens einen Reformer (2) und dem mikrobiellen Reaktor (3) angeordnet ist,
wobei die Trennvorrichtung (16) ausgelegt ist, um eine ungeeignete Substanz, die zum Bilden des Gases in dem mikrobiellen Reaktor (3) ungeeignet ist, von dem durch den mindestens einen Reformer (2) hydrolysierten Abfall zu trennen.

5. Abfallbehandlungssystem (1) nach einem der Ansprüche 1 bis 4,
wobei die mindestens eine Dampferzeugungsvorrichtung (4) eine erste Dampferzeugungsvorrichtung (4A) und eine zweite Dampferzeugungsvorrichtung (4B) beinhaltet, und
wobei das Abfallbehandlungssystem (1) derart ausgelegt ist, dass der Dampf in mindestens entweder der ersten Dampferzeugungsvorrichtung (4A) oder der zweiten Dampferzeugungsvorrichtung (4B) gemäß dem Dampfbedarf von dem Reformer (2) erzeugt wird.

6. Abfallbehandlungssystem (1) nach einem der Ansprüche 1 bis 5,
wobei der mindestens eine Reformer (2) eine Vielzahl von Reformern (2A, 2B) beinhaltet, und
wobei mindestens zwei der Vielzahl von Reformern (2A, 2B) ausgelegt sind, um eine Hydrolyse zu unterschiedlichen Zeitpunkten durchzuführen.

7. Abfallbehandlungssystem (1) nach Anspruch 6, wobei die Vielzahl von Reformern (2A, 2B) einen ersten Reformer (2A) und einen zweiten Reformer (2B) beinhaltet,
wobei das Abfallbehandlungssystem (1) ferner ein Abdampfverteilungsrohr (26) umfasst, die den ersten Reformer (2A) und den zweiten Reformer (2B) verbindet,
wobei der Abdampf nach dem Hydrolysieren des Abfalls von dem ersten Reformer (2A) durch das Abdampfverteilungsrohr (26) dem zweiten Reformer (2B) zugeführt wird, und
wobei der zweite Reformer (2B) durch die Wärme des von dem ersten Reformer (2B) zugeführten Abdampfs erwärmt wird.

8. Abfallbehandlungssystem (1) nach einem der Ansprüche 1 bis 7,
wobei Tr/Tk ≥ 0,3 erfüllt ist, wobei Tr ein Erwärmungszeitraum zum Erhöhen der Temperatur in dem Reformer (2) mit dem Dampf auf eine vorbestimmte Solltemperatur ist und Tk ein Haltezeitraum ist, während dessen die Temperatur in dem Reformer (2) mit dem Dampf (S1) auf der Solltemperatur beibehalten wird.

9. Abfallbehandlungssystem (1) nach Anspruch 8, wobei Tr/Tk ≥ 1,0 erfüllt ist.

10. Abfallbehandlungssystem (1) nach Anspruch 1,
wobei die Feuchtigkeitsmengenerfassungsvorrichtung (30) ausgelegt ist, um die in dem Reformer (2) enthaltene Feuchtigkeitsmenge basierend auf der dem Reformer (2) zugeführten Dampfmenge, der Zeit, in der der Dampf dem Reformer (2) zugeführt wird, und dem Ausmaß der Temperaturänderung in dem Reformer (2) zu erfassen.

11. Abfallbehandlungssystem nach einem der Ansprüche 1 bis 10, wobei die Feuchtigkeitsregulierungsvorrichtung (24) ferner ein in dem Wassereinspritzrohr (24) vorgesehenes Regulierungsventil (32) umfasst, und wobei das Regulierungsventil (32) ausgelegt ist, um einen basierend auf der durch die Feuchtigkeitsmengenerfassungsvorrichtung (30) erfassten Feuchtigkeitsmenge geregelten Öffnungsgrad aufzuweisen.

## Revendications

1. Système de traitement de déchets (1), comprenant :
au moins un reformeur (2) pour l'hydrolyse de déchets avec de la vapeur (S1) ;
un réacteur microbien (3) pour la dégradation microbienne d'une matière reformée contenant au moins un solide des déchets hydrolysés par l'au moins un reformeur (2) ;
au moins un dispositif de génération de vapeur (4) pour la génération de la vapeur (S1) en utilisant uniquement l'énergie de combustion d'un gaz produit dans le réacteur microbien (3) ;
un dispositif d'acquisition de quantité d'humidité (30) pour l'acquisition de la quantité d'humidité contenue dans le reformeur (2) ;
un dispositif d'ajustement d'humidité (24), disposé entre l'au moins un reformeur (2) et le réacteur microbien (3), pour l'ajustement de la teneur en humidité des déchets hydrolysés par l'au moins un reformeur (2), sur la base de la quantité d'humidité acquise par le dispositif d'acquisition de quantité d'humidité (30) ;
un dispositif de déshydratation (14) pour la déshydratation d'un résidu (X2) qui subsiste après que le réacteur microbien dégrade par voie microbienne la matière reformée,
dans lequel le dispositif d'ajustement d'humidité (24) inclut un conduit d'injection d'eau (24) conçu pour alimenter, avec de l'eau déshydratée, qui est déshydratée à partir du résidu par le dispositif de déshydratation (14), la matière reformée tandis que la matière reformée est en train d'être envoyée du reformeur au réacteur microbien (3).

2. Système de traitement de déchets (1) selon la revendication 1, comprenant en outre un conduit de vapeur à rétention de chaleur (8) raccordant le reformeur (2) et le réacteur microbien (3),
dans lequel de la vapeur d'échappement après hydrolyse des déchets alimente, à partir du reformeur (2), le réacteur microbien (3) par le biais de la conduite de vapeur à rétention de chaleur (8), et
dans lequel le réacteur microbien (3) est maintenu au sein d'une plage de températures prédéterminée grâce à la chaleur de la vapeur d'échappement alimentée à partir du reformeur (2).

3. Système de traitement de déchets (1) selon la revendication 1 ou 2, comprenant en outre :
un dispositif de séchage (13) pour le séchage d'un résidu obtenu après que le réacteur microbien (3) dégrade par voie microbienne la matière reformée ; et
un conduit de vapeur de séchage (15) raccordant le reformeur (2) et le dispositif de séchage (13),
dans lequel de la vapeur d'échappement après hydrolyse des déchets alimente, à partir du reformeur (2), le dispositif de séchage (13) par le biais de la conduite de vapeur de séchage (15), et
dans lequel le dispositif de séchage (13) est configuré pour sécher le résidu grâce à la chaleur de la vapeur d'échappement alimentée à partir du reformeur (2).

4. Système de traitement de déchets (1) selon l'une quelconque des revendications 1 à 3, comprenant en outre un dispositif de séparation (16) disposé entre l'au moins un reformeur (2) et le réacteur microbien (3),
dans lequel le dispositif de séparation (16) est configuré pour séparer une substance inappropriée qui est inappropriée pour la production du gaz dans le réacteur microbien (3) à partir des déchets hydrolysés par l'au moins un reformeur (2).

5. Système de traitement de déchets (1) selon l'une quelconque des revendications 1 à 4,
dans lequel l'au moins un dispositif de génération de vapeur (4) inclut un premier dispositif de génération de vapeur (4A) et un second dispositif de génération de vapeur (4B), et
dans lequel le système de traitement de déchets (1) est configuré de telle sorte que la vapeur soit générée dans au moins l'un parmi le premier dispositif de génération de vapeur (4A) ou le second dispositif de génération de vapeur (4B) selon la demande en vapeur du reformeur (2).

6. Système de traitement de déchets (1) selon l'une quelconque des revendications 1 à 5,
dans lequel l'au moins un reformeur (2) inclut une pluralité de reformeurs (2A, 2B), et dans lequel au moins deux de la pluralité de reformeurs (2A, 2B) sont configurés pour réaliser une hydrolyse à des moments différents.

7. Système de traitement de déchets (1) selon la revendication 6, dans lequel la pluralité de reformeurs (2A, 2B) inclut un premier reformeur (2A) et un second reformeur (2B),
dans lequel le système de traitement de déchets (1) comprend en outre un conduit de distribution de vapeur d'échappement (26) raccordant le premier reformeur (2A) et le second reformeur (2B),
dans lequel de la vapeur d'échappement après hydrolyse des déchets alimente, à partir du premier reformeur (2A), le second reformeur (2B) par le biais du conduit de distribution de vapeur d'échappement (26), et
dans lequel le second reformeur (2B) est chauffé par la chaleur de la vapeur d'échappement alimentée à partir du premier reformeur (2B)

8. Système de traitement de déchets (1) selon l'une quelconque des revendications 1 à 7,
dans lequel Tr/Tk ≥ 0,3 est satisfait, où Tr est une période de chauffage pour l'élévation de la température dans le reformeur (2) jusqu'à une température de consigne prédéterminée avec la vapeur, et Tk est une période de maintien pendant laquelle la température dans le reformeur (2) est maintenue à la température de consigne avec la vapeur (S1).

9. Système de traitement de déchets (1) selon la revendication 8, dans lequel Tr/Tk ≥ 1,0 est satisfait.

10. Système de traitement de déchets (1) selon la revendication 1,
dans lequel le dispositif d'acquisition de quantité d'humidité (30) est configuré pour acquérir une quantité d'humidité contenue dans le reformeur (2), sur la base de la quantité de vapeur ayant alimenté le reformeur (2), de la durée pendant laquelle la vapeur a alimenté le reformeur (2) et de la quantité de variation de température dans le reformeur (2).

11. Système de traitement de déchets selon l'une quelconque des revendications 1 à 10, dans lequel le dispositif d'ajustement d'humidité (24) inclut en outre une vanne de régulation (32) ménagée dans le conduit d'injection d'eau (24), et dans lequel la vanne de régulation (32) est configurée pour présenter un degré d'ouverture ajusté sur la base de la quantité d'humidité acquise par le dispositif d'acquisition de quantité d'humidité (30).
